(19) Europäisches Patentamt
European Patent Office
Office européen des brevets

(11) Publication number: **0 344 313 B1**

(12) # EUROPEAN PATENT SPECIFICATION

(45) Date of publication of patent specification: **11.05.94**

(51) Int. Cl.[5]: **G01N 21/77**, G01N 21/78, G01N 21/76, G01N 33/22, C08F 8/42

(21) Application number: **88901922.0**

(22) Date of filing: **19.02.88**

(86) International application number: **PCT/JP88/00174**

(87) International publication number: **WO 88/06287 (25.08.88 88/19)**

(54) **PROBE FOR MEASURING CONCENTRATION OF DISSOLVED GAS.**

(30) Priority: **20.02.87 JP 37284/87**

(43) Date of publication of application: **06.12.89 Bulletin 89/49**

(45) Publication of the grant of the patent: **11.05.94 Bulletin 94/19**

(84) Designated Contracting States: **BE DE FR GB IT**

(56) References cited:

| | |
|---|---|
| **GB-A- 2 132 348** | **JP-A- 5 392 195** |
| **JP-A- 5 470 898** | **JP-A- 5 987 343** |
| **JP-A- 6 063 464** | **JP-A- 6 212 853** |
| **JP-A- 6 230 959** | **JP-A-50 158 681** |
| **JP-A-51 128 394** | **JP-A-51 137 782** |
| **JP-A-54 105 598** | **JP-U- 5 754 055** |
| **JP-U-59 142 761** | **US-A- 4 106 909** |
| **US-A- 4 127 506** | **US-A- 4 200 110** |

**EXTENDED ABSTRACTS, vol. 86-2, 19th - 24th october 1986, page 875, abstract no.583, Princeton, NJ, US; R.C. MURRAY Jr.: "Development of single-fiber optical sensors for blood gas measurements"**

(73) Proprietor: **TERUMO KABUSHIKI KAISHA**
**No. 44-1, Hatagaya 2-chome,**
**Shibuya-ku**
**Tokyo 151(JP)**

(72) Inventor: **NAKAMURA, Hideki**
**101 Yodogawa Mansion**
**20-8, Yodogawa-cho**
**Fujinomiya-shi, Shizuoka-ken 418(JP)**
Inventor: **KANEKO, Masao**
**Rikagaku Kenkyusho**
**2-1, Hirosawa**
**Wako-shi Saitama-ken 351-01(JP)**

(74) Representative: **VOSSIUS & PARTNER**
**Postfach 86 07 67**
**D-81634 München (DE)**

**EP 0 344 313 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

Field of the Invention

The present invention relates to a probe for measurement of dissolved gas concentration for use in optically measuring a concentration of a dissolved gas in water or an aqueous medium such as blood utilizing quenching with the dissolved gas of emission generated by excitation of light.

Disclosure of the Prior Art

Heretofore, so-called electrochemical methods using an oxygen electrode have mainly been used for gas concentration measurement, particularly oxygen concentration measurement in an aqueous medium. However, in measurement of real time, for example, in case of monitoring of oxygen partial pressure in the blood circulating outside the body at use of an artificial lung, such electrochemical methods had many technical problems that, for example, response speed is low, they are susceptible to ambient electromagnetic noise, and their miniaturization is difficult, for example, for use in measuring oxygen concentration in blood with insertion of a catheter into blood vessel. Thus, there have been proposed various devices for measurement of oxygen concentration utilizing a phenomenon that emission such as fluorescence or phosphorescence is quenched with oxygen.

R.C. Murray describes in "Development of Single-Fiber Optical Sensors for Blood Gas Measurements", Extended Abstracts, vol. 86-2, Oct. 19-24th 1986, page 875, abstract No. 583, Princeton, NJ, US the use of pyrene dibutyric acid as sensing material, in a solution of dimethyl formamide.

GB-A-213 23 48 relates to a sensor apparatus comprising a luminescent material incorporated in a carrier material which is relatively permeable to oxygen and relatively impermeable to interfering quenchers. As luminescent material preferably ruthenium(II) complexes with $\alpha$-diimine ligands are employed, wherein the complex is protected by being immobilized in a gas permeable, solvent impermeable polymer, such as a silicon rubber.

Japanese Patent Publication for Opposition Purpose No. 59-24379 and Japanese Patent Unexamined Published Application (hereinafter referred to as J.P. KOKAI) No. 59-108958 (corresponding to EP-A-0 109 959 being published on May 30, 1984) disclose that a silicone polymer is used in matrix containing a fluorescence or phosphorescence-generating substance, namely an emitter. These devices have, in fact, a high sensitivity in measuring oxygen concentration in a gas. However, since it is difficult to uniformly disperse a water soluble emitter such as tris(2,2'-bipyridine)ruthenium (II) complex or 1-pyrenebutyric acid having an excellent property in measurement of oxygen concentration in an aqueous medium and to fix the emitter thereto in order to avoid its elution over a long time, it has been difficult to utilize such a device for measurement of oxygen concentration in an aqueous medium. J.P. KOKAI No. 59-108957 discloses that a polymer containing a plasticizer is used as matrix. However, this device has disadvantages that when blood is to be tested, the plasticizer is liable to elute in the blood and at the same time the emitter elutes to lower the sensitivity, and further, the method of making this device is complicated. Further, J.P. KOKAI No. 59-500896 discloses a device using a porous carrier as a support of the emitter. However, the method of making this device is complicated, and moreover, the device has a complicated structure such as a water impermeable wrapping and is improper for miniaturization.

SUMMARY OF THE INVENTION

An object of the present invention is to provide a probe for measurement of dissolved gas concentration, which is easily made, has a simple structure and thus is easily miniaturized, and wherein, when it is immersed in a test solution, elution of the emitter is remarkably prevented and thus a good response is stably kept over a long time and the problems of the known techniques have been solved.

The above object of the present invention can be attained by a probe for measurement of a dissolved gas concentration in an aqueous medium which comprises a support; a layer provided on the support and comprising a water-soluble polymeric carrier which contains a water-soluble emitter emitting light when excited by an exciting light and having a property that the degree of emission changes by contact with dissolved gas molecules and which is capable of diffusing dissolved gas molecules; and an outside layer covering the carrier layer so as to prevent elution of the emitter and the carrier into the aqueous medium and being permeable to the dissolved gas and water, wherein the carrier is a polymer which is soluble or

swellable in water, hot water or an aqueous electrolyte solution.

BRIEF DESCRIPTION OF THE DRAWING

Figure 1 is a schematic drawing exemplifying a method of measuring a dissolved gas concentration in a liquid using the probe of the present invention, an Figure 2 is an enlarged view of part A in Figure 1.

1 quartz plate (support),
2 carrier layer,
3 outside layer,
4 test solution (water),
5 quartz cell for fluorescence with ground stopper,
6 exciting light,
7 emission,
8 detection

Figures 3 to 6 are drawings indicating the relation of oxygen partial pressure and Io/I observed when the probes of Examples 1 to 4 are used, respectively.

Figure 7 is a sectional view of the tip of the probe of Example 5 of the present invention, and Figure 8 is a schematic view of an example of an optical device in which the probe of Figure 7 is used.

9 optical fiber,
10 plane-convex lens,
11 objective lens for microscope,
12 interference filter,
13 dichroic mirror,
14 extra-high pressure mercury lamp,
15 parallel luminous flux application device,
16 extra-high pressure mercury lamp-lightning device,
17 monochrometor (spectroscope),
18 photomultiplier,
19 high voltage power source for photomultiplier
20 amplifier,
21 oxygen/nitrogen mixed gas

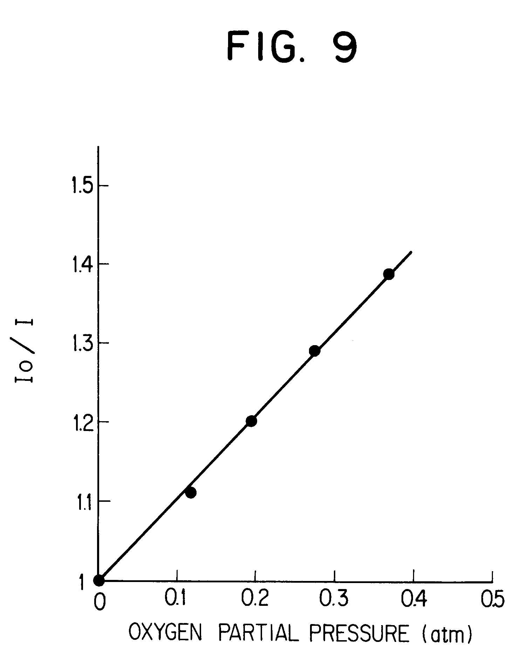

Figure 9 is a drawing indicating the relation of oxygen partial pressure and Io/I observed when the probe of Example 5 is used.

DETAILED DESCRIPTION OF THE INVENTION

Probes of the present invention for measurement of a dissolved gas concentration are advantageously used for measurement of a concentration of gas such as oxygen, nitrogen, hydrogen, carbon dioxide, chlorine or argon dissolved in an aqueous medium such as water or blood.

Any support can be used in the present invention so long as it transmits an exciting light for exciting the emitter, and fluorescence or phosphorescence emitted by excitation of the emitter. Examples of such support include glass, quartz, plastics (for example, polymethyl methacrylate), etc.

Carriers used in the present invention are desirably those which are synthetic polymers such as polyvinylpyrrolidone, polyvinyl alcohol, polyoxyethylene, polyacrylic acid and its salts, polymethacrylic acid and its salts, polyacrylamide, poly(hydroxyethyl methacrylate), polyvinylpyridine, polyvinylbipyridine and polyallylamine, and copolymers of a monomer composing these polymers and other one or more monomers, or natural high molecular compounds such as starches and gelatin; which are soluble or swellable in water, hot water or aqueous electrolyte solutions; and which are colorless and high in light permeability.

Emitters, i.e. substances which are excited by an exciting light and emit fluorescence or phosphorescence are desirably selected from the group consisting of tris(2,2'-bipyridine)ruthenium (II) complex, tris-(1,10'-phenanthroline)ruthenium (II) complex and mixtures thereof. Other desirable emitters include 1-pyrenebutyric acid and its salts. Measurement of a dissolved gas concentration can be carried out with good sensitivity by using such an emitter. Further, the emitter can be mixed with the carrier in an aqueous solution owing to high water solubility thereof so that the emitter can uniformly be dispersed in the carrier. Other usable emitters include pyrene, 1-aminopyrene, perylene, perylene dibutyrate, 2,7-dichlorofluoresceine, etc. A material prepared by firmly binding such an emitter to the carrier through a chemical bond, for example as a copolymer may also be used.

As the outside layer permeable to a dissolved gas, hardened silicone polymers are most desirable. Silicone polymers have good permeability to dissolved gas, particularly oxygen and steam and are chemically inactive, have excellent stability and are very readily formed into a membrane. There can be used as silicone polymers, for example, commercially available room temperature-hardened type silicones (for example, Silicone RTV and Silicone SE 5001 made by Toray Silicone Co., Ltd.), copolymers of silicone, polycarbonate and urethane, etc. In addition, there can also be used cellulose dialysis membrane, porous polypropylene, porous polyethylene, membranes having dissolved gas permeability and having hydrophilic groups such as styrene-vinylpyrrolidone copolymer, which, however, require techniques such as adhesion in preparation of a membrane on the aforesaid carrier. If a dissolved gas is oxygen, it has been well known that oxygen molecule $O_2$ has an action quenching emission such as fluorescence or phosphorescence generated by light excitation. In this connection, a quenching action means a phenomenon that emission intensity decreases in the presence of oxygen compared with in the absence of oxygen. The following relation (referred to as Stern-Volmer equation) is usually established between emission intensity and oxygen concentration:

$$I_o / I = 1 + K_q \cdot \tau_o [O_2] \qquad (1)$$

wherein Io is emission intensity in the substantial absence of oxygen molecules, I is emission intensity when oxygen concentration is $[O_2]$, $K_q$ is quenching reaction rate constant and $\tau$o is emission life in the substantial absence of oxygen molecule.

Since the value of $K_q \cdot \tau$o is thought to be constant, oxygen concentration can be determined by measurement of Io and I.

However, when oxygen concentration is measured using a fixed emitter, it is necessary for the carrier layer containing the emitter to have a high oxygen diffusion coefficient and to dissolve oxygen therein.

In the present invention, a probe for measurement of a dissolved gas concentration is composed of a combination of a membrane having dissolved gas permeability and a carrier containing an emitter. When oxygen gas is referred to as an example, oxygen molecules easily pass through this oxygen-permeable membrane and move into the carrier from the test solution. Although many carriers (typically represented by polyvinyl alcohol) act as an oxygen-impermeable membrane in a dry state, the carrier in the present invention is made wet by permeated water and therefore, oxygen molecules dissolve promptly in the carrier and diffuse with a high diffusion coefficient. The emitter molecules are excited to an excitation state by light h$\nu$ from the exciting light source. When an excited emitter molecule does not collide with oxygen molecule, the emitter molecule go back to ground state being accompanied by emission h$\nu$' (emission life at this time is $\tau$o). On the other hand, when an emitter molecule in an excited state collides with oxygen molecule, some energy or electrons are transferred from the emitter molecule to the oxygen molecule, and the emitter molecule goes back to the ground state without emission. This is a quenching reaction. Since the oxygen concentration in the carrier is proportional to oxygen concentration in the test solution, it can be determined according to equation (1) after measurement of emission intensity. That is, the values of $K_q$ and $\tau$o are in advance determined by measuring the emission intensity Io when the oxygen concentration is O and the emission intensity I on a test solution having a known oxygen concentration and using equation (I), and then emission intensity on a test solution having an unknown oxygen concentration is determined to obtain an oxygen concentration of the test solution.

In determination of oxygen concentration, sensitivity becomes higher as values of $k_q$ and $\tau$o become higher, and thus use of an emitter having a long $\tau$o value is desirable. It is also desirable in this sense that the emitter in the present invention is selected from tris(2,2'-bipyridine)ruthenium (II) complex, and 1-pyrenebutyric acid and its salts.

The outside layer in the present invention has several functions. That is, the membrane (i.e. the outside layer) introduces the dissolved gas molecules from the test solution to the carrier, and at the same time inhibits outflow of the carrier itself into the test solution and also prevents lowering of sensitivity owing to elution of the emitter.

A probe for measurement of a dissolved gas concentration in the present invention can be composed using a support such as a quartz plate as indicated in Figure 1, or can also be prepared in an extremely small size, for example by providing a carrier layer and an outside layer on the tip of an optical fiber.

A device for measurement of a dissolved gas concentration can readily be composed by combining a probe for measurement of a dissolved gas concentration of the present invention with an exciting light source for exciting the emitter and a measuring part for detecting the intensity of fluorescence or phosphorescence.

Example 1

Chloride of tris(2,2'-bipyridine)ruthenium (II) complex (a reagent made by Aldrich) was dissolved in deionized water to obtain a 0.1 mM aqueous solution (A). 0.1 g of powder of polyvinyl alcohol (a reagent made by Nakarai Chemical, polymerization degree of about 2000, saponification degree of 99% or more) was placed in a test tube, 1 ml of the aforesaid aqueous solution (A) was added thereto, and the powder was dissolved therein with stirring on a water bath of 90 °C to obtain about 10% aqueous polyvinyl alcohol solution (B) containing 1 mM tris(2,2'-bipyridine)ruthenium (II) complex.

The above aqueous solution (B) was added dropwise and applied onto quartz plate 1 (thickness 1 mm, length 40 mm, width 8 mm) horizontally placed, and then dried by allowing to stand it in a constant temperature bath of 60 °C for about one hour to form fixing layer 2 of polyvinyl alcohol having the thickness of about 10 micrometers and containing tris(2,2'-bipyridine)ruthenium(II) complex. The central part (a rectangle of 20 mm long and 5 mm wide) of this carrier layer 2 was alone left over and the other parts were cut off.

A room temperature-hardened type silicone (made by Toray Silicone Co., Ltd., SE5001, clear type) was thinly applied further onto this quartz plate so that it covers the above fixing layer. The resulting quartz plate was allowed to stand in a constant temperature bath of 60 °C for about 3 hours to provide outside layer 3 (about 0.2 mm thick) comprising a hardened silicone resin.

Quartz plate 1 coated with carrier layer 2 and outside layer 3 was fixed using a silicone rubber-made tool in quartz cell for fluorescence with ground stopper 5 (made by Fujiwara Seisakusho Co., Ltd., optical path length: 10 mm, optical path width: 10 mm, all surfaces: transparent) so that the width direction of quartz plate 1 is toward diagonal direction, namely it is 45 ° against the light incidence window.

Water was injected inside the cell until the outside layer was completely immersed, argon gas (purity 99.99%) was introduced therein through a capillary at a flow rate of 50 ml/min for 15 minutes, and then the cell was quickly closely plugged. The cell was set in a fluorescent spectrophotometer (made by Nippon Bunko Co., Ltd., FP 550A) and emission intensity Io was measured at an exciting light wavelength of 460 nm and an emission side spectroscope wavelength of 595 nm.

Separately, oxygen-argon mixed gases having various oxygen partial pressure were prepared using the flow ratio mixing method, and introduced into the cell in the same manner as abovementioned at a flow rate of 50 ml/min for 15 minutes, and the cell was closely plugged. Emission intensity I was then measured for each oxygen partial pressure under the same condition as in argon gas.

Plotting of the relations between Io/I and oxygen partial pressure in a graph revealed good straight line relations having an intercept of Io/I = 1 as shown in Figure 3.

Further, similar experiments carried out after preservation in water under the air at 37 °C for 10 days revealed no change in the slope of the straight line.

Incidentally, oxygen concentration in water is proportional to oxygen partial pressure of the introduced mixed gas (Henry's law).

Example 2

The procedure in Example 1 was repeated except for use of polyvinylpyrrolidone (made by Wako Pure Chemicals Co., Ltd., K-30 for cosmetics) in place of polyvinyl alcohol to form on a quartz plate a fixing layer comprising polyvinylpyrrolidone containing tris(2,2'-bipyridine) ruthenium (II) complex and form further thereon an outside layer of the hardened silicone resin. Relations between oxygen partial pressure and Io/I as determined in the same manner as in Example 1 revealed good straight line relations, as shown in Figure 4.

Example 3

The procedure in Example 1 was repeated except for use of chloride of tris(1,10'-phenanthroline)-ruthenium (II) complex in place of chloride of tris(2,2'-bipyridine)ruthenium (II) complex to form on a quartz plate a carrier layer comprising polyvinyl alcohol containing this complex and an outside layer comprising the silicone resin. The determined change of Io/I by oxygen partial pressure revealed good relations as shown in Figure 5.

In this connection 450 nm was adopted as the exciting light wavelength of the fluorescent spectrophotometer, and 590 nm as the emission side spectroscope wavelength.

Example 4

A predetermined amount of 1-pyrenebutyric acid (made by Aldrich Co., reagent) was dissolved in 10 mM aqueous sodium hydroxide solution to prepare an aqueous solution (D) containing 0.1 mM 1-pyrenebutyric acid and 10 mM sodium hydroxide.

Then, 0.1 g of powder of sodium polyacrylate (made by Wako Pure Chemicals Co., Ltd., reagent) was added to 1 ml of this aqueous solution (D) to make a solution. A carrier layer comprising sodium polyacrylate containing 1-pyrenebutyric acid was provided on a quartz plate using this aqueous solution in the same manner as in Examples 1 to 3. An outside layer comprising a hardened silicone resin was provided thereon, and relations between oxygen partial pressure and Io/I were determined in the same manner as in Examples 1 to 3 except for use of 342 nm as exciting light wavelength of the fluorescent spectrophotometer and 395 nm as emission side spectroscope wavelength. As a result, good straight line relations were obtained as shown in Figure 6.

Example 5

The ruthenium complex pendant type N-vinylpyrrolidone copolymer having the following structural formula was synthesized according to the known technique (Journal of Polymer Science 20, 593, 1982).

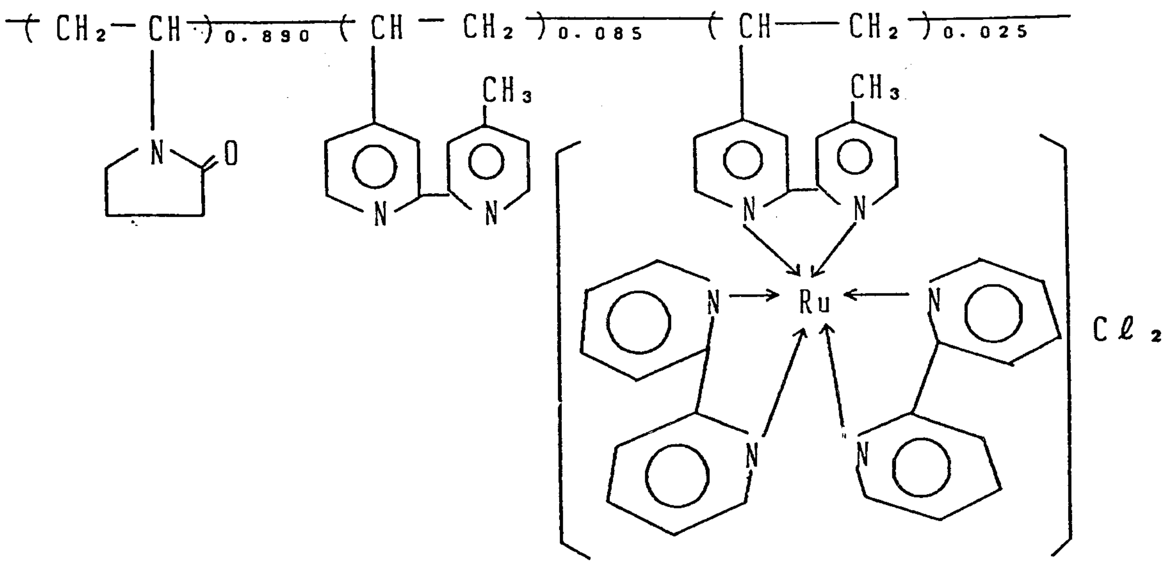

The copolymer, wherein the emitter and the carrier are chemically bound, was as a material for the fixing layer. A thin coating of the copolymer was formed on one of the exposed core end surfaces of a plastic-made optical fiber (2 m) (made by Mitsubishi Rayon Co., Ltd., SK-10, outside diameter 2.5 mm), and an outside layer comprising a silicone-polycarbonate-urethane terpolymer (made by Toyo Polymer Co., Ltd., TR-1352-I) and having a thickness of about 50 µm was provided thereon. The resulting material was used as a probe for measurement of oxygen concentration (Figure 7).

The other of the core end surfaces of the fiber was bound to an optical apparatus whose schematic drawing is shown in Figure 8.

Voltage value in proportion to emission intensity as obtained from amplifier 20 was read, and relations between Io/I and oxygen partial pressure were plotted in a graph in the same manner as in Examples 1 to 4, whereby a good correlation was obtained as shown in Figure 9.

Oxygen/nitrogen mixed gas was used in place of oxygen/argon mixed gas in Examples 1 to 4.

Although the present examples refer to cases wherein oxygen gas was used as a dissolved gas, good straight line relations can also be obtained when other dissolved gases are used such as nitrogen gas, hydrogen gas, carbon dioxide gas, chlorine gas and argon gas by combination of an emitter having a property that degree of emission by application of light changes by contact with particular dissolved gas molecules.

EFFECT OF THE INVENTION

Devices for measurement of a dissolved gas concentration of the present invention, wherein a water soluble high molecular compound is used as a carrier containing an emitter, and an outside layer being permeable to a dissolved gas is provided between the carrier and a test solution, give excellent straight line relations between emission intensity ratio (Io/I) and partial pressure of the dissolved gas, have a good response to dissolved gas concentration, and further have a stable performance that changes in sensitivity are extremely small even after long time immersion in water. Further, when an exciting light and an emission both in visible range are used, it is possible to use a ultraviolet light-impermeable material such as polymethyl methacrylate as a medium of light (for example, optical fiber).

In spite of having thus high performances, probes for measurement of a dissolved gas concentration of the present invention have a simple structure and can thus easily be miniaturized. For example, application of the probes to measurement at a minute site such as in blood vessel is possible by forming a carrier layer and an outside layer on the tip of optical fiber. Further, an organic solvent, plasticizer and the like harmful on a living body are not used at all in the preparation steps, and thus the present probes are excellent in safety and can be readily clinically applied.

**Claims**

**1.** A probe for measurement of a dissolved gas concentration in an aqueous medium which comprises a support; a layer provided on the support and comprising a water-soluble polymeric carrier which contains a water-soluble emitter emitting light when excited by an exciting light and having a property that the degree of emission changes by contact with dissolved gas molecules and which is capable of diffusing the dissolved gas molecules; and an outside layer covering the carrier layer so as to prevent elution of the emitter and the carrier into the aqueous medium and being permeable to the dissolved gas and water, wherein the carrier is a polymer which is soluble or swellable in water, hot water or an aqueous electrolyte solution.

**2.** The probe for measurement of a dissolved gas concentration of claim 1 wherein the carrier is a member selected from the group consisting of polyvinylpyrrolidone, polyvinyl alcohol, polyoxyethylene, polyacrylic acid and its salts, polymethacrylic acid and its salts, polyacrylamide, poly(hydroxyethyl methacrylate), polyvinylpyridine, polyvinylbipyridine and polyallylamine, and copolymers of a monomer composing these polymers and other one or more monomers, starch, and gelatin.

**3.** The probe for measurement of a dissolved gas concentration of claim 1 wherein the emitter is selected from the group consisting of tris(2,2'-bipyridine) ruthenium (II) complex, tris(1,10'-phenanthroline)-ruthenium (II) complex and mixtures thereof.

**4.** The probe for measurement of a dissolved gas concentration of claim 1 wherein the water-soluble emitter is 1-pyrenebutyric acid or a salt thereof.

**5.** The probe for measurement of a dissolved gas concentration of claim 1 wherein the outside layer comprises a hardened silicone polymer.

**Patentansprüche**

**1.** Sonde zum Messen der Konzentration eines gelösten Gases in wäßrigem Medium, die einen Stützträger, eine Schicht, bereitgestellt auf dem Stützträger, welche einen wasserlöslichen polymeren Träger umfaßt, der einen wasserlöslichen Emitter enthält, welcher Licht aussendet, wenn er durch Anregungslicht angeregt wird, und bei welchem die Möglichkeit gegeben ist, daß sich der Grad der Emission beim Kontakt mit gelösten Gasmolekülen ändert und welcher imstande ist, gelöste Gasmoleküle eindiffundieren zu lassen, und eine Außenschicht umfaßt, die die Trägerschicht bedeckt, um die Elution von Emitter und Träger in das wäßrige Medium zu verhindern, und die durchlässig ist für gelöstes Gas und Wasser, wobei der Träger ein Polymer ist, welches in Wasser, heißem Wasser oder einer wäßrigen Elektrolytlösung löslich oder quellbar ist.

**2.** Sonde zum Messen der Konzentration eines gelösten Gases nach Anspruch 1, wobei der Träger aus der Gruppe ausgewählt wird, die aus Polyvinylpyrrolidon, Polyvinylalkohol, Polyoxyethylen, Polyacrylsäure und ihren Salzen, Polymethacrylsäure und ihren Salzen, Polyacrylamid, Poly(hydroxyethylmethacrylat), Polyvinylpyridin, Polyvinylbipyridin und Polyallylamin, sowie Copolymeren eines Monomers aus diesen Polymeren und einem oder mehreren anderen Monomeren, Stärke und Gelatine besteht.

**3.** Sonde zum Messen der Konzentration eines gelösten Gases nach Anspruch 1, wobei der Emitter aus der Gruppe ausgewählt wird, die aus Tris(2,2'-bipyridin)ruthenium(II)-Komplex, Tris(1,10'-phenanthrolin)-ruthenium(II)-Komplex und Gemischen davon besteht.

**4.** Sonde zum Messen der Konzentration eines gelösten Gases nach Anspruch 1, wobei der wasserlösliche Emitter 1-Pyren-Buttersäure oder ein Salz davon ist.

**5.** Sonde zum Messen der Konzentration eines gelösten Gases nach Anspruch 1, wobei die Außenschicht ein gehärtetes Silikonpolymer umfaßt.

**Revendications**

**1.** Capteur pour la mesure d'une concentration de gaz dissous dans un milieu aqueux qui comprend un support ; une couche appliquée sur le support et comprenant un véhicule polymère soluble dans l'eau qui contient un émetteur soluble dans l'eau émettant de la lumière lorsqu'il est excité par une lumière d'excitation et ayant la propriété que le degré d'émission varie par contact avec des molécules de gaz dissous et qui est capable de diffuser les molécules de gaz dissous ; et une couche extérieure recouvrant la couche de véhicule de manière à éviter l'élution de l'émetteur et du véhicule dans le milieu aqueux et perméable au gaz dissous et à l'eau, dans lequel le véhicule est un polymère qui est soluble ou gonflable dans l'eau, l'eau chaude ou une solution aqueuse d'électrolyte.

**2.** Capteur pour la mesure d'une concentration de gaz dissous selon la revendication 1, dans lequel le véhicule est choisi parmi la polyvinylpyrrolidone, l'alcool polyvinylique, le polyoxyéthylène, l'acide polyacrylique et ses sels, l'acide polyméthacrylique et des sels, le polyacrylamide, le polyméthacrylate d'hydroxyéthyle, la polyvinylpyridine, le polyvinylbipyridine et la polyallylamine et les copolymères d'un monomère composant ces polymères et d'un ou plusieurs autres monomères, l'amidon et la gélatine.

**3.** Capteur pour la mesure d'une concentration de gaz dissous selon la revendication 1, dans lequel l'émetteur est choisi parmi le complexe tris(2,2'-bipyridine)ruthénium (II), le complexe tris(1,10'-phénanthroline)ruthénium (II) et leurs mélanges.

**4.** Capteur pour la mesure d'une concentration de gaz dissous selon la revendication 1, dans lequel l'émetteur soluble dans l'eau est l'acide 1-pyrène-butyrique ou un de ses sels.

**5.** Capteur pour la mesure d'une concentration de gaz dissous selon la revendication 1, dans lequel la couche extérieure comprend un polymère de silicone durci.

FIG. 1

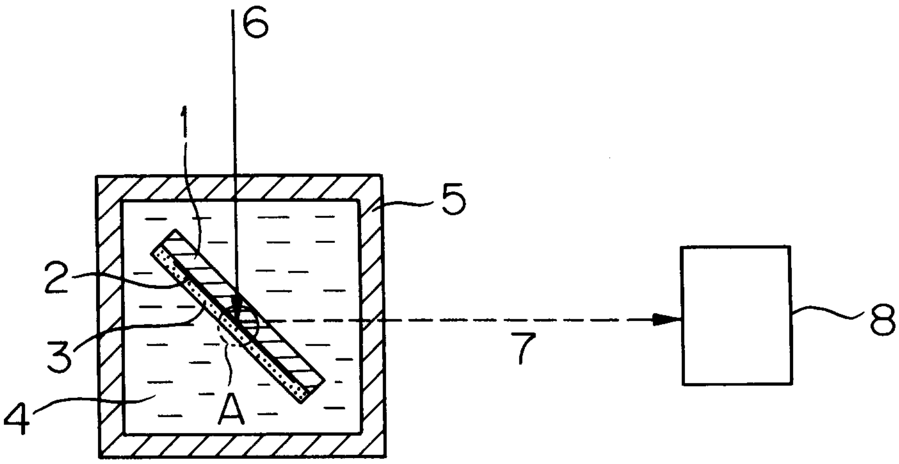


FIG. 2

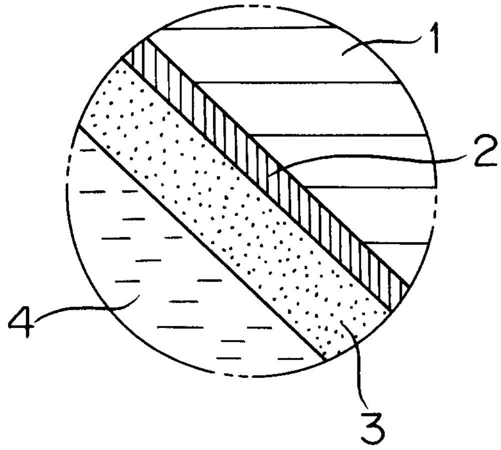

FIG. 3

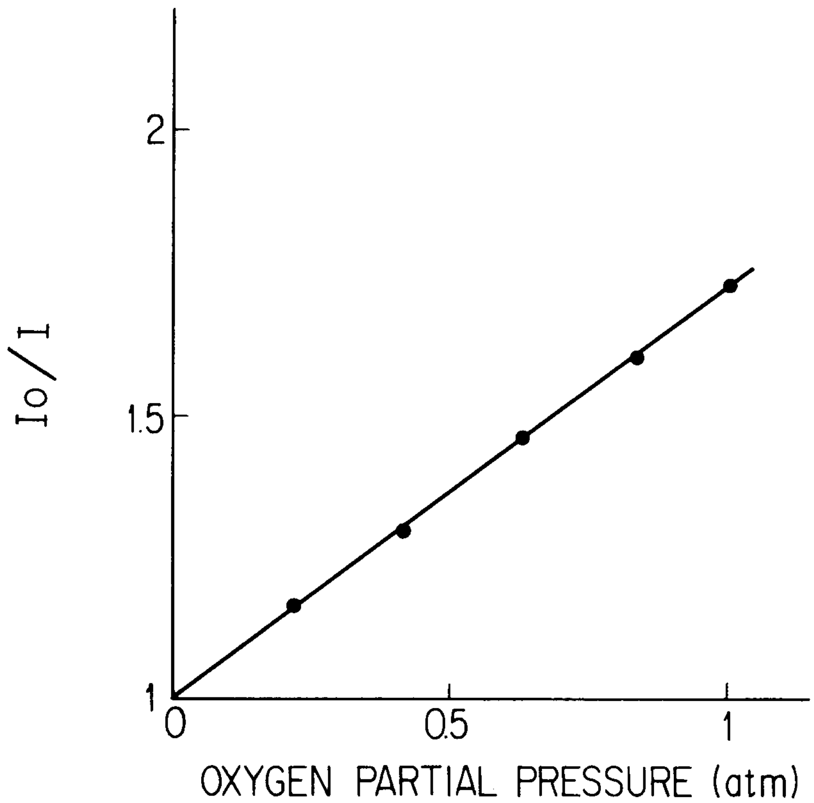
2
1.5
1
Io/I
0
0.5
1
OXYGEN PARTIAL PRESSURE (atm)

FIG. 4

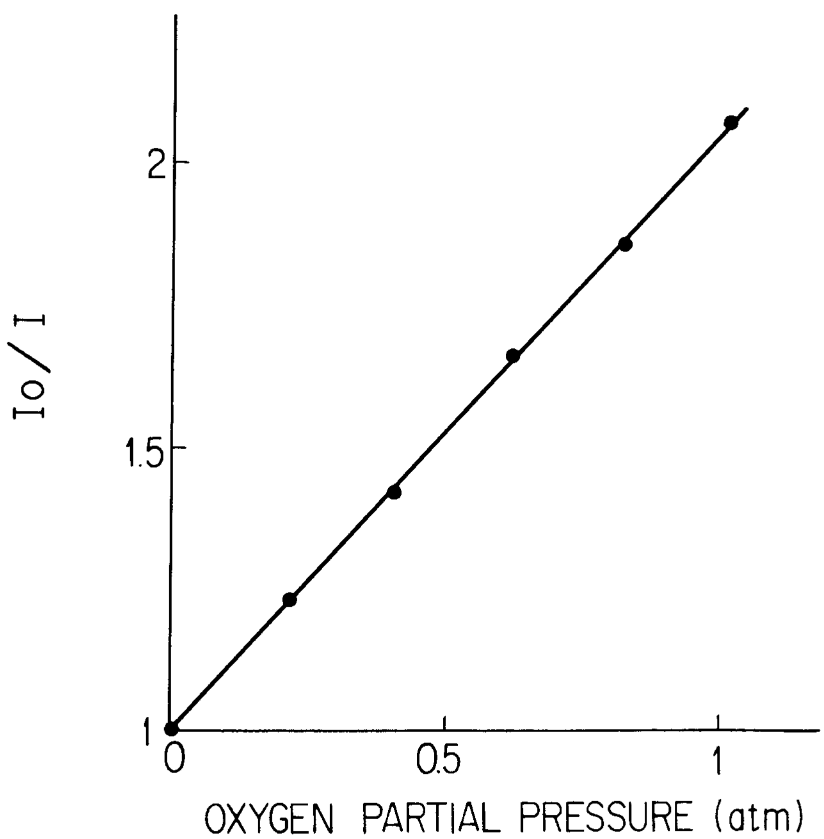
2
1.5
1
Io/I
0
0.5
1
OXYGEN PARTIAL PRESSURE (atm)

FIG. 5

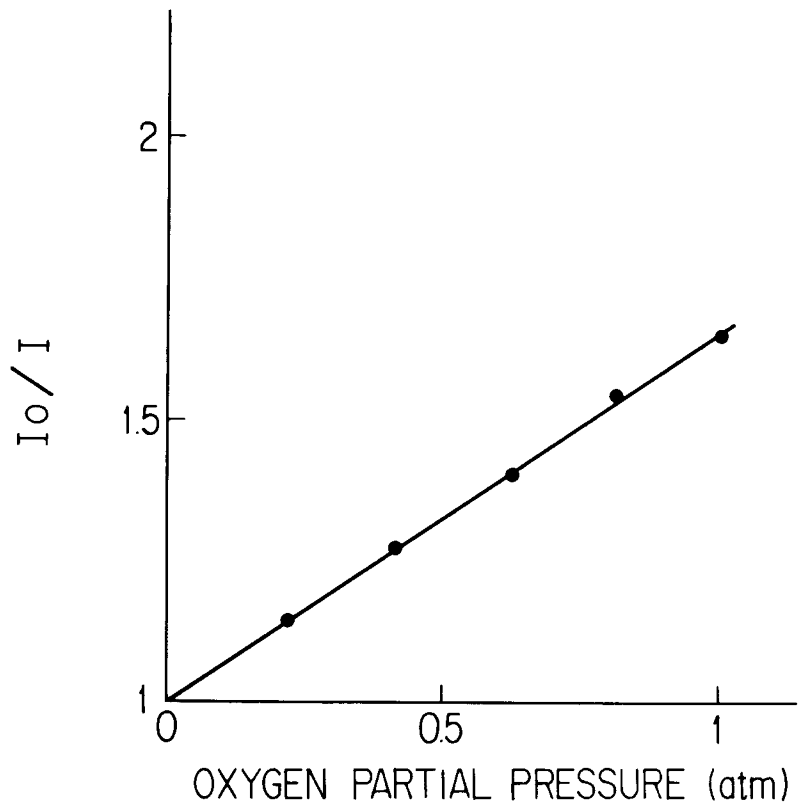
Io/I
2
1.5
1
0
0.5
1
OXYGEN PARTIAL PRESSURE (atm)

FIG. 6

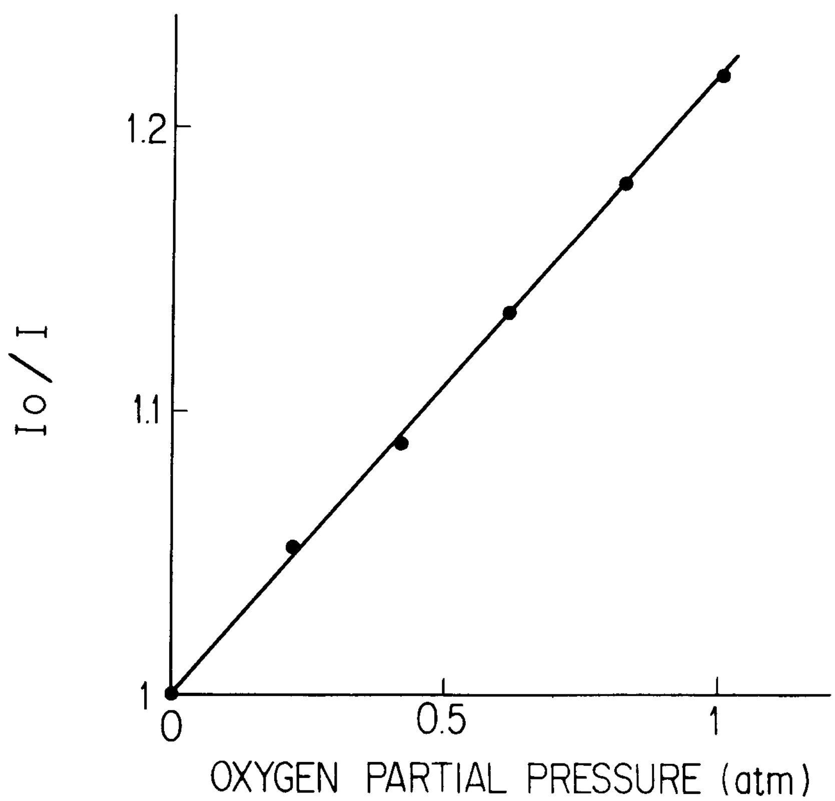
Io/I
1.2
1.1
1
0
0.5
1
OXYGEN PARTIAL PRESSURE (atm)

FIG. 7

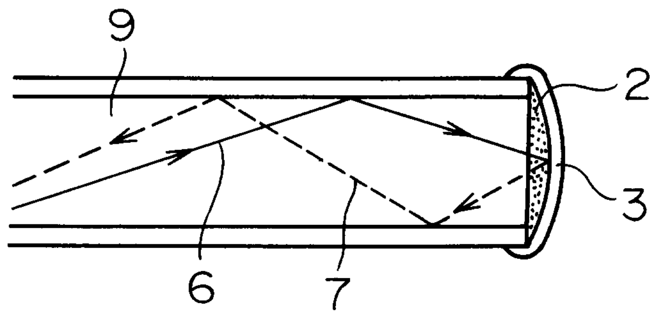


FIG. 8

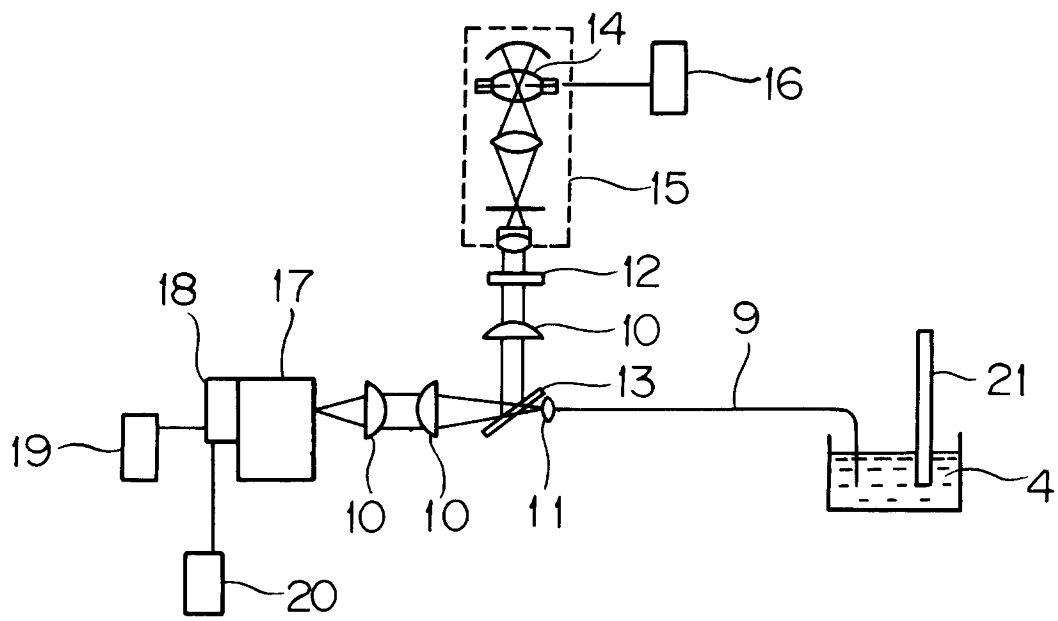

FIG. 9